Europäisches Patentamt
European Patent Office
Office européen des brevets

(11) Veröffentlichungsnummer: 0 284 954
A2

(12) # EUROPÄISCHE PATENTANMELDUNG

(21) Anmeldenummer: 88104545.4

(51) Int. Cl.4: C07D 501/46 , A61K 31/545

(22) Anmeldetag: 22.03.88

(30) Priorität: 03.04.87 DE 3711343

(43) Veröffentlichungstag der Anmeldung:
05.10.88 Patentblatt 88/40

(84) Benannte Vertragsstaaten:
AT BE CH DE ES FR GB GR IT LI NL SE

(71) Anmelder: BAYER AG
Konzernverwaltung RP Patentabteilung
D-5090 Leverkusen 1 Bayerwerk(DE)

(72) Erfinder: Dornhagen, Jürgen Dr.
Jurastrasse 2
D-7859 Eimeldingen(DE)
Erfinder: Angerbauer, Rolf Dr.
Sterntalerweg 29
D-5600 Wuppertal 1(DE)
Erfinder: Metzger, Karl Georg Dr.
Pahlkestrasse 75
D-5600 Wuppertal 1(DE)
Erfinder: Zeiler, Hans-Joachim Dr.
Elsbeekerstrasse 46
D-5620 Velbert 15(DE)

(54) **Cephalosporinderivate, Verfahren zu ihrer Herstellung und ihre Verwendung in Arzneimitteln.**

(57) Die Erfindung betrifft neue Cephalosporinderivate der Formel (I)

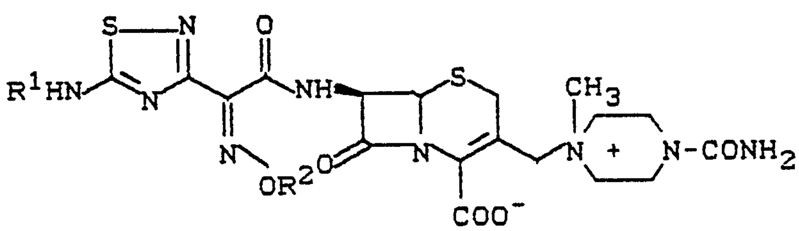

in welcher $R^1$ und $R^2$ die in der Beschreibung angegebene Bedeutung haben, Verfahren zu ihrer Herstellung sowie ihre Verwendung als Arzneimittel, insbesondere in der antibakteriellen Therapie.

EP 0 284 954 A2

## Cephalosporinderivate, Verfahren zu ihrer Herstellung und ihre Verwendung in Arzneimitteln

Die Erfindung betrifft Cephalosporinderivate, Verfahren zu ihrer Herstellung sowie ihre Verwendung als Arzneimittel, insbesondere in der antibakteriellen Therapie.

Cephalosporine die als Acyl-Seitenkette einen 2-(2-Aminothioazol-4-yl)-2-alkoxyiminoessigsäurerest und in 3-Stellung einen (4-Aminocarbonyl-1-methylpiperazinium)methylrest tragen sind aus DE-A1 35.06.159 bekannt.

Die vorliegende Erfindung betrifft neue Cephalosporinderivate der allgemeinen Formel (I)

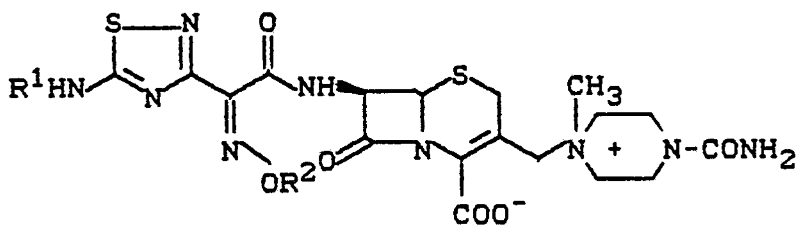

in welcher

$R^1$ -für Wasserstoff oder
-für eine Aminoschutzgruppe steht
und
$R^2$ -für Alkyl,
-für Cycloalkyl, oder
-für Alkenyl steht.

Alkyl steht im allgemeinen für einen geradkettigen oder verzweigten Kohlenwasserstoffrest mit 1 bis 12 Kohlenstoffatomen. Bevorzugt wird Niederalkyl mit 1 bis etwa 6 Kohlenstoffatomen. Beispielsweise seien Methyl, Ethyl, Propyl, Isopropyl, Butyl, Isobutyl, Pentyl, Isopentyl, Hexyl, Isohexyl, Heptyl, Isoheptyl, Octyl und Isooctyl genannt.

Alkenyl steht im allgemeinen für einen geradkettigen oder verzweigten Kohlenwasserstoffrest mit 2 bis 12 Kohlenstoffatomen und einer oder mehreren, bevorzugt mit einer oder zwei Doppelbindungen. Bevorzugt ist der Niederalkylrest mit 2 bis etwa 6 Kohlenstoffatomen und einer Doppelbindung. Besonders bevorzugt ist ein Alkenylrest mit 2 bis 4 Kohlenstoffatomen und einer Doppelbindung. Beispielsweise seien Allyl, Propenyl, Isopropenyl, Butenyl, Isobutenyl, Pentenyl, Isopentenyl, Hexenyl, Isohexenyl, Heptenyl, Isoheptenyl, Octenyl und Isooctenyl genannt.

Aminoschutzgruppe im Rahmen der oben angegebenen Definition steht im allgemeinen für eine in der β-Lactam-Chemie üblichen Schutzgruppe aus der Reihe: 4-Methoxy phenyl, 4-Methoxymethyloxyphenyl, 4-[(2-Methoxyethoxy)methyloxy]phenyl, 3,4-Dimethoxyphenyl, Benzyl, 2-Nitrobenzyl, 4-Nitrobenzyl, 4-Methoxybenzyl, 2,4-Dimethoxybenzyl, 3,4-Dimethoxybenzyl, 2,4,6-Trimethoxybenzyl, Vinyl, Allyl, tert-Butoxycarbonyl, Benzyloxycarbonyl, 2-Nitrobenzyloxycarbonyl, 4-Nitrobenzyloxycarbonyl, Formyl, Acetyl, Chloracetyl, Trichloracetyl, Trifluoracetyl, Benzoyl, Methoxycarbonyl, Allyloxycarbonyl, 2,4-Dimethoxybenzyloxycarbonyl, 2,2-Diethoxyethyl, Methoxycarbonylmethyl, tert-Butoxycarbonylmethyl, Allyloxymethyl, Benzoylmethyl, Bis-(4-methoxyphenyl)methyl, Methoxymethyl, Methylthiomethyl, Methoxyethoxymethyl, 2-(Methylthiomethoxy)-ethoxycarbonyl, 2-Hydroxy-2-phenylmethyl, Methoxy-(4-methoxyphenyl)methyl, Trimethyl-, Triethyl-, Triphenylsilyl, tert-Butyl-dimethylsilyl, tert-Butyl-diphenylsilyl, [2-(Trimethylsilyl)ethoxy]methyl.

Bevorzugte Verbindungen der allgemeinen Formel (I) sind solche,
in welchen

$R^1$ -für Wasserstoff, oder
-für Acetyl steht
und
$R^2$ -für geradkettiges oder verzweigtes Alkyl mit bis zu 8 Kohlenstoffatomen steht, oder
-für Cyclopropyl, Cyclopentyl oder Cyclohexyl steht, oder
-für geradkettiges oder verzweigtes Alkenyl mit bis zu 8 Kohlenstoffatomen und ein oder zwei Doppelbindungen steht.

Besonders bevorzugt sind solche Verbindungen der allgemeinen Formel (I), in welchen

$R^1$ -für Wasserstoff steht

und

$R^2$ -für geradkettiges oder verzweigtes Alkyl mit bis zu 6 Kohlenstoffatomen,

-für Cyclopentyl, Cyclohexyl oder

-für geradkettiges oder verzweigtes Alkenyl mit bis zu 4 Kohlenstoffatomen und einer Doppelbindung steht.

Von den erfindungsgemäßen Verbindungen der allgemeinen Formel (I) können mehrere Isomeren entstehen, welche alle antibiotisch wirksam sind und bei Bedarf durch Chromatographie oder Kristallisation getrennt werden können.

Die Verbindungen der allgemeinen Formel (I) erhält man, indem Carbonsäuren der allgemeinen Formel (II)

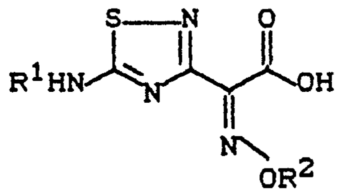

(II)

in welcher

$R^1$ und $R^2$ die oben angegebene Bedeutung haben,

nach Aktivierung der Carboxylgruppe durch Überführung in ein gemischtes Anhydrid, beispielsweise mit Chlorameisensäureethylester oder Methansulfonsäurechlorid, oder durch Überführen in das Säurehalogenid, oder durch Überführen in einen aktivierten Ester mit beispielsweise N-Hydroxybenztriazol und Cyclohexylcarbodiimid,

mit der β-Lactam-Verbindung der Formel (III)

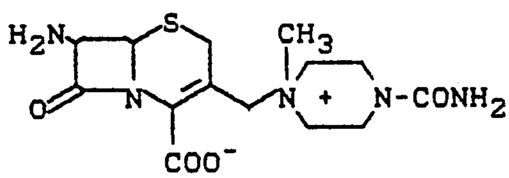

(III)

umgesetzt werden, dann gegebenenfalls Schutzgruppen abgespalten werden und die gewünschten Salze oder aus Salzen die freien Betaine hergestellt werden.

Für die Kupplung von Carbonsäuren (II) an die β-Lactam-Verbindung (III) kann eine große Zahl von aus der Cephalosporin-bzw. Penicillinchemie bekannten Methoden verwendet werden. Es hat sich als vorteilhaft erwiesen, die Carbonsäuren der allgemeinen Formel (II) ohne Aminschutzgruppe zu aktivieren und dann mit der β-Lactam-Verbindung der Formel (III), die als Salze mit einem Amin in Lösung gebracht wurden, zu kuppeln.

Besonders vorteilhaft ist die Aktivierung mit Sulfonsäurederivaten der allgemeinen Formel (IV) zu Anhydriden der allgemeinen Formel (V), wie im folgenden Reaktionsschema verdeutlicht wird:

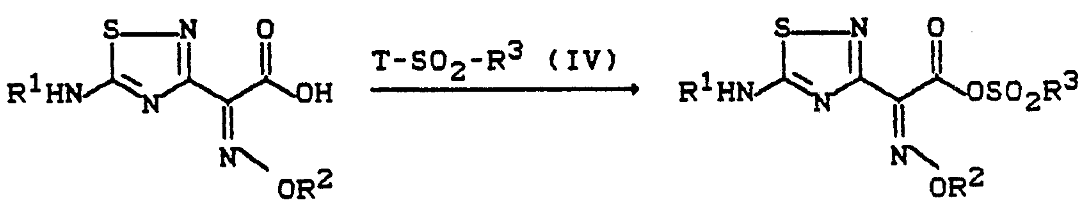


(II) (V)

Hierbei steht in der Formel (IV) bzw. (V)

T - für den Rest $R^3$-$SO_2$-O-oder Halogen

und

$R^3$ -für Alkyl mit bis zu 10 Kohlenstoffatomen, das gegebenenfalls substituiert ist durch Fluor, Chlor, Cyano, Alkyl, Alkoxycarbonyl, Alkoxy oder Alkyl mit jeweils bis zu 4 Kohlenstoffatomen, oder

-für Phenyl, das gegebenenfalls substituiert ist durch Fluor, Chlor, Brom, Cyano, Alkyl, Alkoxy, Alkylthio, Alkoxycarbonyl mit jeweils bis zu 4 Kohlenstoffatomen, Nitro, Trifluormethyl oder Phenyl.

Wenn $R^3$ substituiert ist, sind bevorzugt 1 bis 3 Substituenten, besonders bevorzugt die oben genannten vorhanden.

Ganz besonders bevorzugt stellt $R^3$ einen Methyl-oder p-Tolylrest dar.

Die gemischten Anhydride der allgemeinen Formel (V) werden hergestellt, indem man die Carbonsäuren der allgemeinen Formel (II) und 1 bis 1,4 Äquivalente eines Amins in einem Lösemittel löst und mit 1 bis 1,2 Äquivalenten eines Sulfonsäurederivates der Formel (IV) reagieren läßt.

Als Lösemittel eignen sich alle Solventien, die sich unter den Reaktionsbedingungen nicht verändern. Hierzu gehören bevorzugt Ether wie beispielsweise Diethylether, Dioxan oder Tetrahydrofuran, oder Chlorkohlenwasserstoffe wie Methylenchlorid, Chloroform oder Tetrachlormethan, oder Amide wie Dimethylformamid oder Hexamethylphosphorsäuretriamid, oder Acetonitril oder Aceton. Ebenso ist es möglich, Gemische der genannten Lösemittel einzusetzen.

Als Amine eignen sich tertiäre Amine, wie beispielsweise Triethylamin, Ethyl-diisopropylamin oder Tributyl amin, aber auch sterisch gehinderte sekundäre Amine, wie beispielsweise Diisopropylamin. Ebenso können Gemische der genannten Amine eingesetzt werden.

Die Umsetzungen können bei Temperaturen zwischen -80°C und Raumtemperatur durchgeführt werden. Vorteilhaft wird die Aktivierung mit Methansulfonsäurechlorid in Dimethylformamid bei -40°C bis -60°C innerhalb von 0,2 bis 24 Stunden, vorzugsweise 0,5 bis 5 Stunden durchgeführt.

Zum Lösen der $\beta$-Lactam-Verbindung der Formel (III) können die bei der Herstellung der Verbindungen der Formel (V) genannten Lösemittel oder Wasser, sowie als Base die dort genannten Amine verwendet werden.

Besonders vorteilhaft ist auch eine Aktivierung der Carbonsäuren der allgemeinen Formel (II) durch Überführung in einen aktivierten Ester mit beispielsweise N-Hydroxysuccinimid und Dicyclohexylcarbodiimid oder mit 1-Hydroxybenztriazol und Dicyclohexylcarbodiimid.

Als Lösemittel eignen sich hierbei alle Solventien, die sich auch für die Herstellung von Anhydriden der allgemeinen Formel (V) eignen und dort bereits aufgeführt sind.

Die Umsetzungen können bei Temperaturen zwischen -30°C und +100°C durchgeführt werden. Vorteilhaft wird mit 1-Hydroxybenztriazol und Dicyclohexylcarbodiimid in Dimethylformamid bei Raumtemperatur 2 bis 6 Stunden akti viert, dann vom ausgefallenen Dicyclohexylharnstoff abgesaugt und mit der $\beta$-Lactam-Verbindung der Formel (III) in Form einer Lösung ihres Aminsalzes innerhalb von 2 bis 24 Stunden umsetzt. Zum Lösen der $\beta$-Lactam-Verbindung der Formel (III) können die bei der Herstellung der Verbindungen der Formel (V) genannten Lösemittel sowie als Base die dort genannten Amine verwendet werden.

Die $\beta$-Lactam-Verbindung der Formel (III) ist aus DE-A1 35 06 159 bekannt.

Die Verbindungen der allgemeinen Formel (II) sind bekannt oder können nach bekannten Methoden hergestellt werden [GB-20 94 794; DE-A-35 12 225].

Alternativ zu dem oben beschriebenen Verfahren können die erfindungsgemäßen Stoffe der allgemeinen Formel (I) auch hergestellt werden, indem man

Ester der allgemeinen Formel (VI)

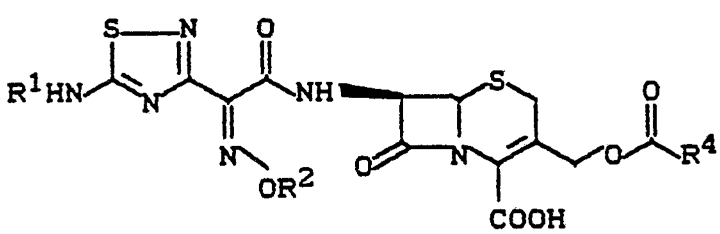

(VI)

in welcher

$R^1$ und $R^2$ die angegebene Bedeutung haben

und

$R^4$ -für gegebenenfalls substituiertes Alkyl oderAryl steht, bevorzugt für Methyl, Ethyl, Propyl, Chlormethyl, Dichlormethyl, Trichlormethyl, Trifluormethyl oder Phenyl steht, oder besonders bevorzugt für eine Methylgruppe steht.
silyliert,
die Silylverbindung der allgemeinen Formel (VII)

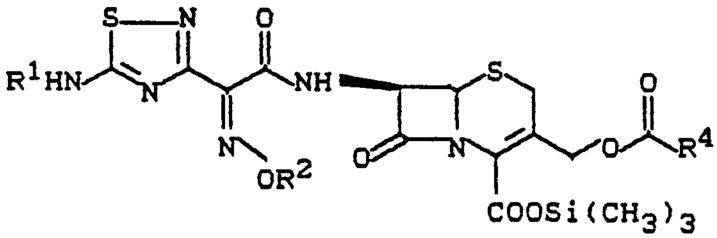

(VII)

in welcher
R', $R^2$ und $R^4$ die oben angegebene Bedeutung haben,
in die Iodmethylverbindungen der allgemeinen Formel (VIII)

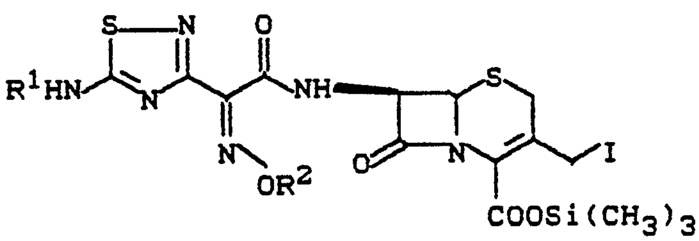

(VIII)

in welcher
R' und $R^2$ die oben angegebene Bedeutung haben,
überführt
und diese anschließend mit 1-Aminocarbonyl-4-methylpiperazin der Formel (IX)

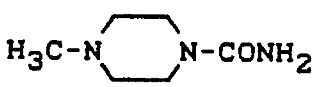

(IX)

umsetzt.

Im folgenden Schema soll dieses Verfahren verdeutlicht werden:

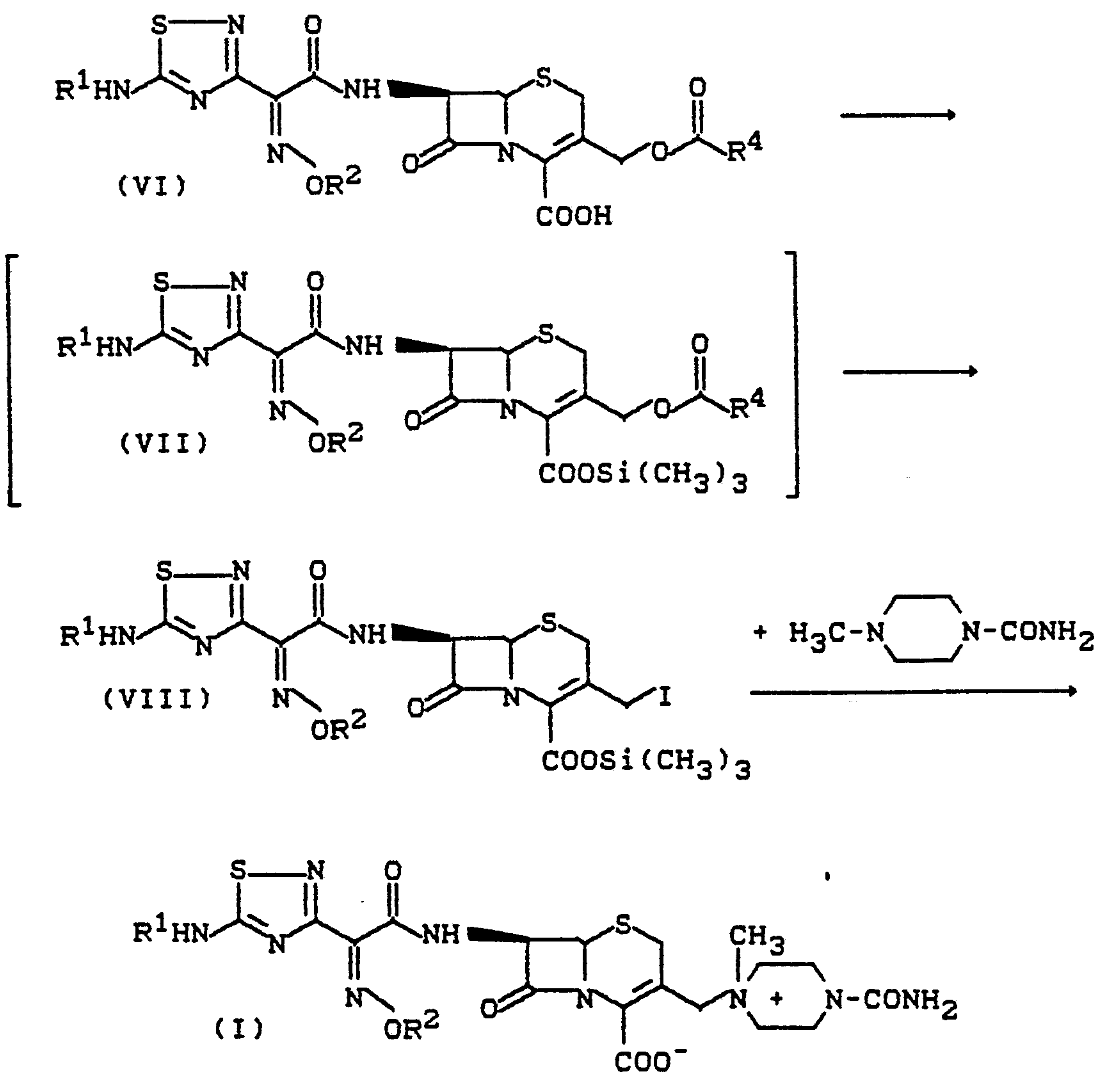

(VI)

(VII)

(VIII)

(I)

Hierzu werden die Ausgangsverbindungen der allgemeinen Formel (VI) in einem geeigneten organischen Lösemittel suspendiert und durch Silylierung zu den Silylestern (VIII) in Lösung gebracht. Besonders geeignete organische Lösemittel sind Chlorkohlenwasserstoffe wie beispielsweise Methylenchlorid, Chloroform oder Tetrachlorethan. Die Silylierung wird mit einem üblichen Silylierungsmittel wie beispielsweise Trimethylchlorsilan (TMCS), Hexamethyldi-silazan (HMDS), N,O-Bis(trimethylsilyl)acetamid (BSA), N,O-Bis-(trimethylsilyl)trifluoracetamid (BSTFA), N-Methyl-N-trimethylsilylacetamid (MSA), N-Methyl-N-trimethylsilyltrifluoracetamid (MSTFA), 1,3-Bis(trimethylsilyl)harnstoff oder Trimethylsilyltrifluormethansulfonat durchgeführt. Dabei können auch mehrere Silylierungsmittel im Gemisch eingesetzt werden.

Die Silylierung erfolgt im allgemeinen in einem Temperaturbereich von -30°C bis +70°C, bevorzugt von -10°C bis +10°C innerhalb von 5 bis 30 Minuten. Vorteilhaft wird ein bis zu zehnfacher Überschuß des Silylierungsmittels eingesetzt, bevorzugt ein zwei bis fünffacher Überschuß.

Die so erhaltene Lösung des Trimethylsilylesters der Formel (VII) wird in einem Temperaturbereich von -40°C bis +30°C, bevorzugt mit 3 bis 4 Äquivalenten eines Trialkylsilyliodids, besonders bevorzugt Trimethylsilyliodid, innerhalb von 15 Minuten bis 2 Stunden, bevorzugt innerhalb von 30 Minuten bis 1 Stunde zu Verbindungen der allgemeinen Formel (VIII) umgesetzt.

Die Verbindungen der Formel (VIII) werden vorteilhaft nicht isoliert, sondern direkt ohne Reinigung mit 1-Aminocarbonyl-4-methylpiperazin der Formel (IX) umgesetzt.

Die erfindungsgemäßen Verbindungen sind gegen ein sehr breites Spektrum von Mikroorganismen wirksam. Mit ihrer Hilfe können gram-negative und gram-positive Bakterien und bakterienähnliche Mikroorganismen bekämpft, sowie die durch diese Erreger hervorgerufenen Erkrankungen verhindert, gebessert und/oder geheilt werden.

Besonders wirksam sind die erfindungsgemäßen Verbindungen gegen Bakterien und bakterienähnliche

Mikroorganismen. Sie sind daher besonders gut zur Prophylaxe und Chemotherapie von lokalen und systemischen Infektionen in der Human-und Tiermedizin geeignet, die durch diese Erreger hervorgerufen werden.

Beispielsweise können lokale und/oder systemische Erkrankungen behandelt und/oder verhindert werden, die durch die folgenden Erreger oder durch Mischungen der folgenden Erreger verursacht werden: Gram-positive Kokken, z.B. Staphylokokken (Staph. aureus, Staph. epidermidis) und Streptokokken (Strept. agalactiae, Strept. faecalis, Strept. pneumoniae, Strept. pyogenes); gram-negative Kokken (Neisseria gonorrhoeae) sowie gram-negative Stäbchen wie Enterobakteriaceen, z.B. Escherichia coli, Hämophilus influenzae, Citrobacter (Citrob. freundii, Citrob. divernis), Salmonella und Shigella; ferner Klebsiellen (Klebs. pneumoniae, Klebs. oxytoca), Enterobacter (Ent. aerogenes, Ent. agglomerans), Hafnia, Serratia (Serr. marcescens), Proteus (Pr. mirabilis, Pr. rettgeri, Pr. vulgaris), Providencia, Yersinia, sowie die Gattung Acinetobacter. Darüber hinaus umfaßt das antibakterielle Spektrum die Gattung Pseudomonas (Ps. aeruginosa, Ps. maltophilia) sowie strikt anaerobe Bakterien wie z.B. Bacteroides fragilis, Vertreter der Gattung Peptococcus, Peptostreptococcus sowie die Gattung Clostridium; ferner Mykoplasmen (M. pneumoniae, M. hominis, M. urealyticum) sowie Mykobakterien, z.B. Mycobacterium tuberculosis.

Die obige Aufzählung von Erregern ist lediglich beispielhaft und keineswegs beschränkend aufzufassen. Als Krankheiten, die durch die genannten Erreger oder Mischinfektionen verursacht und durch die erfindungsgemäßen Verbindungen verhindert, gebessert oder geheilt werden können, seien beispielsweise genannt:
Infektionskrankheiten beim Menschen wie zum Beispiel Otitis, Pharyngitis, Pneumonie, Peritonitis, Pyelonephritis, Cystitis, Endocarditis, Systeminfektionen, Bronchitis (akut, chronisch), septische Infektionen, Erkrankungen der oberen Luftwege, diffuse Panbronchiolitis, pulmonäres Emphysem, Dysenterie, Enteritis, Leberabszesse, Urethritis, Prostatitis, Epididymitis, gastrointestinale Infektionen, Knochen-und Gelenkinfektionen, zystische Fibrose, Hautinfektionen, postoperative Wundinfektionen, Abszesse, Phlegmone, Wundinfektionen, infizierte Verbrennungen, Brandwunden, Infektionen im Mundbereich, Infektionen nach Zahnoperationen, Osteomyelitis, septische Arthritis, Cholecystitis, Peritonitis mit Appendicitis, Cholangitis, intraabdominale Abszesse, Pankreatitis, Sinusitis, Mastoiditis, Mastitis, Tonsillitis, Typhus, Meningitis und Infektionen des Nervensystems, Salpingitis, Endometritis, Genital-Infektionen, Pelveoperitonitis und Augeninfektionen.

Außer beim Menschen können bakterielle Infektionen auch bei anderen Spezies behandelt werden. Beispielhaft seien genannt:
Schwein: Coli-diarrhoe, Enterotoxämie, Sepsis, Dysenterie, Salmonellose, Metritis-Mastitis-Agalaktiae-Syndrom, Mastitis;
Wiederkäuer (Rind, Schaf, Ziege): Diarrhoe, Sepsis, Bronchopneumonie, Salmonellose, Pasteurellose, Mykoplasmose, Genitalinfektionen;
Pferd: Bronchopneumonien, Fohlenlähme, puerperale und postpuerperale Infektionen, Salmonellose;
Hund und Katze: Bronchopneumonie, Diarrhoe, Dermatitis, Otitis, Harnwegsinfekte, Prostatitis;
Geflügel (Huhn, Pute, Wachtel, Taube, Ziervögel und andere): Mycoplasmose, E. coli-Infektionen, chronische Luftwegserkrankungen, Salmonellose, Pasteurellose, Psittakose.

Ebenso können bakterielle Erkrankungen bei der Aufzucht und Haltung von Nutz-und Zierfischen behandelt werden, wobei sich das antibakterielle Spektrum über die vorher genannten Erreger hinaus auf weitere Erreger wie zum Beispiel Pasteurella, Brucella, Campylobacter, Listeria, Erysipelothrix, Corynebakterien, Borellia, Treponema, Nocardia, Rikettsien, Yersinia, erweitert.

Zur vorliegenden Erfindung gehören pharmazeutische Zubereitungen, die neben nicht-toxischen, inerten pharmazeutisch geeigneten Trägerstoffen eine oder mehrere erfindungsgemäße Verbindungen enthalten oder die aus einem oder mehreren erfindungsgemäßen Wirkstoffen bestehen, sowie Verfahren zur Herstellung dieser Zubereitungen.

Zur vorliegenden Erfindung gehören auch pharmazeutische Zubereitungen in Dosierungseinheiten. Dies bedeutet, daß die Zubereitung in Form einzelner Teile, z.B. Tabletten, Dragees, Kapseln, Pillen, Suppositorien und Ampullen vorliegen, deren Wirkstoffgehalt einem Bruchteil oder einem Vielfachen einer Einzeldosis entsprechen. Die Dosierungseinheiten können z.B. 1, 2, 3 oder 4 Einzeldosen oder 1/2, 1/3 oder 1/4 einer Einzeldosis enthalten. Eine Einzeldosis enthält vorzugsweise die Menge Wirkstoff, die bei einer Applikation verabreicht wird und die gewöhnlich einer ganzen, einer halben oder einem Drittel oder einem Viertel einer Tagesdosis entspricht.

Unter nicht-toxischen, inerten pharmazeutisch geeigneten Trägerstoffen sind feste, halbfeste oder flüssige Verdünnungsmittel, Füllstoffe und Formulierungshilfsmittel jeder Art zu verstehen.

Als bevorzugte pharmazeutische Zubereitungen seien Tabletten, Dragees, Kapseln, Pillen, Granulate, Suppositorien, Lösungen, Suspensionen und Emulsionen, Pasten, Salben, Gele, Cremes, Lotions, Puder und Sprays genannt.

Tabletten, Dragees, Kapseln, Pillen und Granulate können den oder die Wirkstoffe neben den üblichen Trägerstoffen enthalten, wie (a) Füll-und Streckmittel, z.B. Stärken, Milchzucker, Rohrzucker, Glukose, Mannit und Kieselsäure, (b) Bindemittel, z.B. Carboxymethylcellulose, Alginate, Gelatine, Polyvinylpyrrolidon, (c) Feuchthaltemittel, z.B. Glycerin, (d) Sprengmittel, z.B. Agar-Agar, Calciumcarbonat und Natriumcarbonat, (e) Lösungsverzögerer, z.B. Paraffin und (f) Resorptionsbeschleuniger, z.B. quarternäre Ammoniumverbindungen, (g) Netzmittel, z.B. Cetyl-alkohol, Glycerinmonostearat, (h) Adsorptionsmittel, z.B. Kaolin und Bentonit und (i) Gleitmittel z.B. Talkum, Calcium-und Magnesiumstearat und feste Polyethylenglykole oder Gemische der unter (a) bis (i) aufgeführten Stoffe.

Die Tabletten, Dragees, Kapseln, Pillen und Granulate können mit den übliche, gegebenenfalls Opakisierungsmitteln enthaltenden, Überzügen und Hüllen versehen sein und auch so zusammengesetzt sein, daß sie den oder die Wirkstoffe nur oder bevorzugt in einem bestimmten Teil des Intestinaltraktes gegebenenfalls verzögert abgeben, wobei als Einbettungsmassen z.B. Polymersubstanzen und Wachse verwendet werden können.

Der oder die Wirkstoffe können gegebenenfalls mit einem oder mehreren der oben angegebenen Trägerstoffe auch in mikroverkapselter Form vorliegen.

Suppositorien können neben dem oder den Wirkstoffen die üblichen wasserlöslichen oder wasserunlöslichen Trägerstoffe enthalten, z.B. Polyethylenglykole, Fette, z.B. Kakaofett und höhere Ester (z.B. $C_{14}$-Alkohol mit $C_{16}$-Fettsäure) oder Gemische dieser Stoffe.

Salben, Pasten, Cremes und Gele können neben dem oder den Wirkstoffen die üblichen Trägerstoffe enthalten, z.B. tierische und pflanzliche Fette, Wachse, Paraffine, Stärke, Tragant, Cellulosederivate, Polyethylenglykole, Silikone, Bentonite, Kieselsäure, Talkum und Zinkoxid oder Gemische dieser Stoffe.

Puder und Sprays können neben dem oder den Wirkstoffen die üblichen Trägerstoffe enthalten, z.B. Milchzucker, Talkum Kieselsäure, Aluminiumhydroxid, Calciumsilikat und Polyamidpulver oder Gemische dieser Stoffe, Sprays können zusätzlich die üblichen Treibmittel, z.B. Chlorfluorkohlenwasserstoffe, enthalten.

Lösungen und Emulsionen können neben dem oder den Wirkstoffen die üblichen Trägerstoffe wie Lösungsmittel, Lösungsvermittler und Emulgatoren, z.B. Wasser, Ethylalkohol, Isopropylalkohol, Ethylcarbonat, Ethylacetat, Benzylalkohol, Benzylbenzoat, Propylenglykol, 1,3-Butylenglykol, Dimethylformamid, Öle, insbesondere Baumwollsaatöl, Erdnußöl, Maiskeimöl, Olivenöl, Ricinusöl und Sesamöl, Glycerin, Glycerinformal, Tetrahydofurfurylalkohol, Polyethylenglykole und Fettsäureester des Sorbitans oder Gemische dieser Stoffe enthalten.

Zur parenteralen Applikation können die Lösungen und Emulsionen auch in steriler und blutisotonischer Form vorliegen.

Suspensionen können neben dem oder den Wirkstoffen die üblichen Trägerstoffe wie flüssige Verdünnungsmittel, z.B. Wasser, Ethylalkohol, Propylenglykol, Suspendiermittel, z.B. ethoxylierte Isostearylalkohole, Polyoxyethylensorbit-und Sorbitan-Ester, mikrokristalline Cellulose, Aluminiummetahydroxid, Bentonit, Agar-Agar und Tragant oder Gemische dieser Stoffe enthalten.

Die genannten Formulierungsformen können auch Färbemittel, Konservierungsstoffe sowie geruchs-und geschmacksverbesserte Zusätze, z.B. Pfefferminzöl und Eukalyptusöl und Süßmittel, z.B. Saccharin, enthalten.

Die therapeutisch wirksamen Verbindungen sollen in den oben aufgeführten pharmazeutischen Zubereitungen vorzugsweise in einer Konzentration von etwa 0,1 bis 99,5, vorzugsweise von etwa 0,5 bis 95 Gew.-%, der Gesamtmischung vorhanden sein.

Die oben aufgeführten pharmazeutischen Zubereitungen können außer den erfindungsgemäßen Verbindungen auch weitere pharmazeutische Wirkstoffe enthalten.

Die Herstellung der oben aufgeführten pharmazeutischen Zubereitungen erfolgt in üblicher Weise nach bekannten Methoden, z.B. durch Mischen des oder der Wirkstoffe mit dem oder den Trägerstoffen.

Die genannten Zubereitungen können bei Mensch und Tier entweder oral, rektal, parenteral (intravenös, intramuskulär, subkutan), intracisternal, intravaginal, intraperitoneal, lokal (Puder, Salbe, Tropfen) und zur Therapie von Infektionen in Hohlräumen, Körperhöhlen angewendet werden. Als geeignete Zubereitungen kommen Injektionlösungen, Lösungen und Suspensionen für die orale Therapie, Gele, Aufgußformulierungen, Emulsionen, Salben oder Tropfen in Frage. Zur lokalen Therapie können ophtalmologische und dermatologische Formulierungen, Silber-und andere Salze, Ohrentropfen, Augensalben, Puder oder Lösungen verwendet werden. Bei Tieren kann die Aufnahme auch über das Futter oder Trinkwasser in geeigneten Formulierungen erfolgen. Ferner können Gele, Pulver, Puder, Tabletten, Retard-Tabletten, Premixe, Konzentrate, Granulate, Pellets, Tabletten, Boli, Kapseln, Aerosole, Sprays, Inhalate bei Mensch und Tier angewendet werden. Ferner können die erfindungsgemäßen Verbindungen in andere Trägermaterialien wie zum Beispiel Kunststoffe, (Kunststoffketten zur lokalen Therapie), Kollagen oder

Knochenzement eingearbeitet werden.

Im allgemeinen hat es sich sowohl in der Human-als auch in der Veterinärmedizin als vorteilhaft erwiesen. den oder die erfindungsgemäßen Wirkstoffe in Gesamtmengen von etwa 0,5 bis etwa 500, vorzugsweise 5 bis 100 mg/kg Körpergewicht je 24 Stunden, gegebenenfalls in Form mehrerer Einzelgaben. zur Erzielung der gewünschten Ergebnisse zu verabreichen. Eine Einzelgabe enthält den oder die erfindungsgemäßen Wirkstoffe vorzugsweise in Mengen von etwa 1 bis etwa 80, insbesondere 3 bis 30 mg/kg Körpergewicht. Es kann jedoch erforderlich sein, von den genannten Dosierungen abzuweichen, und zwar in Abhängigkeit von der Art und dem Körpergewicht des zu behandelnden Objekts, der Art und der Schwere der Erkrankung, der Art der Zubereitung und der Applikation des Arzneimittels sowie dem Zeitraum bzw. Intervall, innerhalb welchem die Verabreichung erfolgt.

So kann es in einigen Fällen ausreichend sein, mit weniger als der obengenannten Menge Wirkstoff auszukommen, während in anderen Fällen die oben angeführte Wirkstoffmenge überschritten werden muß. Die Festlegung der jeweils erforderlichen optimalen Dosierung und Applikationsart der Wirkstoffe kann durch jeden Fachmann aufgrund seines Fachwissens leicht erfolgen.

Die neuen Verbindungen können in den üblichen Konzentrationen und Zubereitungen zusammen mit dem Futter bzw. mit Futterzubereitungen oder mit dem Trinkwasser gegeben werden. Dadurch kann eine Infektion durch gram-negative oder gram-positive Bakterien verhindert, gebessert und/oder geheilt werden und dadurch eine Förderung des Wachstums und eine Verbesserung der Verwertung des Futters erreicht werden.

Herstellungsbeispiele

Beispiel 1

7β-[2-(5-Amino-1,2,4-thiadiazol-3-yl)-2-(Z)-methoxyiminoacetamido]-3-(4-aminocarbonyl-1-methylpiperazinium)methyl-3-cephem-4-carboxylat hydrosulfat

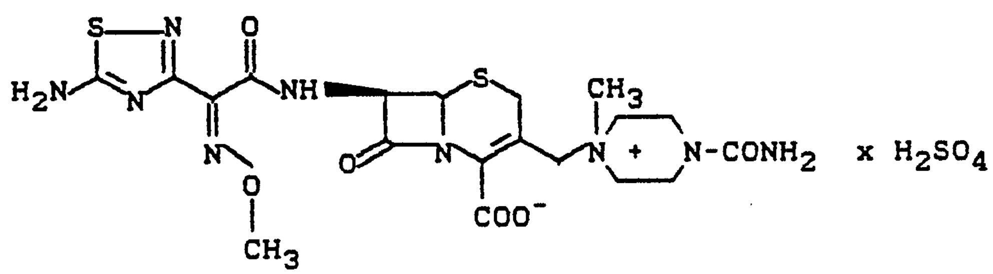

3,0 g (14,8 mmol) 2-(5-Amino-1,2,4-thiadiazol-3-yl)2-(Z)-methoxyiminoessigsäure werden unter Stickstoffatmosphäre bei Raumtemperatur in 30 ml absolutem Dimethylformamid gelöst. Bei -50°C werden 4 ml einer 1:2 molaren Mischung von N-Ethyldiisopropylamin und Tributylamin zugesetzt. 1,1 ml (14,2 mmol) Methansulfonsäurechlorid werden zugegeben und die Lösung wird 30 Minuten bei -50°C gerührt. Anschließend wird diese Lösung schnell zu einer auf 0°C gekühlten Lösung von 3,0g (6,6 mmol) 7-Amino-3-(4-aminocarbonyl-1-methylpiperazinium)methyl-3-cephem-4-carboxylat (x $H_2SO_4$) in 10 ml Wasser und 5 ml Triethylamin gegeben. Nach 15 Minuten wird die Reaktionslösung auf 1,0 l Aceton gegeben. Der entstehende Niederschlag wird abgesaugt und getrocknet. Zur Reinigung wird das Sulfat hergestellt, das in einer Ausbeute von 1,27 g anfällt.

$^1$H-NMR (DCOOD): δ =6,13 [1]d; 5,55 [1]d; 5,12 [1]bd; 4,56 [1]bd, 4,3 - 4,1 [4]bd + s; 4,03 [1] bd, 3,88 -3,57 [8]m, 3,43 [3]s ppm.

$^{13}$C-NMR (DCOOD):δ = 182,36 (C-5"); 167,47; 166,35; 163,04; 162,73 (C-1", C-8, C-9, C-4‴); 153m43 (C-3); 143,90 (C-2"); 136,55 (C-4); 121,07 (C-3); 70,17 (C-3'); 68,04 ($NOCH_3$); 63,16; 62,96 (C-2"); 62,69 (C-7); 61,88 (C-6); 48,69 ($N-CH_3$); 41,04 (C-3‴); 33,30 (C-2).

Beispiel 2

7β-[2-(5-Amino-1,2,4-thiadiazol-3-yl)-2-(Z)-ethoxyiminoacetamido]-3-[4-aminocarbonyl-1-methylpiperazinium)methyl-3-cephem-4-carboxylat

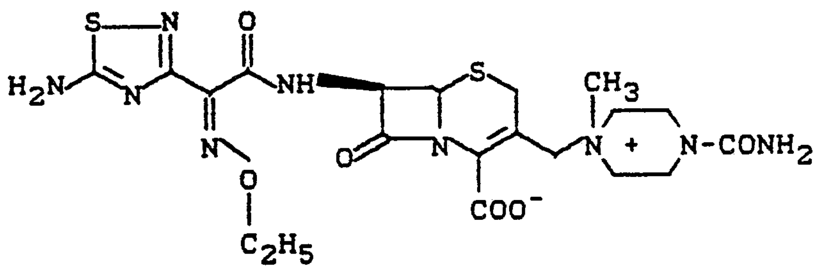

2,16 g (10 mmol) 2-(5-amino-1,2,4-thiadiazol-3-yl)-2-(Z)-ethoxyiminoessigsäure werden unter Stickstoffatmosphäre bei Raumtemperatur in 20 ml absolutem Dimethylformamid gelöst. Nach Zugabe von 2,7 ml (10 mmol) Tributylamin wird auf -50°C gekühlt. 773 µl (10 mol) Methansulfonsäurechlorid werden zugegeben und die Lösung wird 30 Minuten bei -50°C gerührt. Anschließend wird diese Lösung schnell zu einer auf 0°C gekühlten Lösung von 2,3 g (5 mmol) 7-Amino-3-(4-aminocarbonyl-1-methylpiperazinium)methyl-3-cephem-4-carboxylat (x $H_2SO_4$) in 6,7 ml Wasser und 3,4 ml Triethylamin gegeben. Nach 15 Minuten wird die Reaktionslösung auf 1 l Aceton gegeben. Der entstehende Niedeschlag wird abgesaugt, getrocknet und über Adsorberharz HP-20 chromatographiert (Elutionsmittel: $H_2O$ bis $H_2O$/Aceton 80/20, V/V.
Ausbeute: 770 mg
$^1$H-NMR (DCOOD): δ = 6.10 [1]d; 5,50 [1]d; 5,09 [1]bd; 4,50 [1]bd; 4,49 [2]q; 4,5 - 3,63 [12]m; 3,38 [3]s; 1,40 [3]t ppm.

Beispiel 3

7β-[2-(5-Amino-1,2,4-thiadiazol-3-yl)-2-(Z)-propyloxyiminoacetamido]-3-(4-aminocarbonyl-1-methylpiperazinium)methyl-3-cephem-4-carboxylat

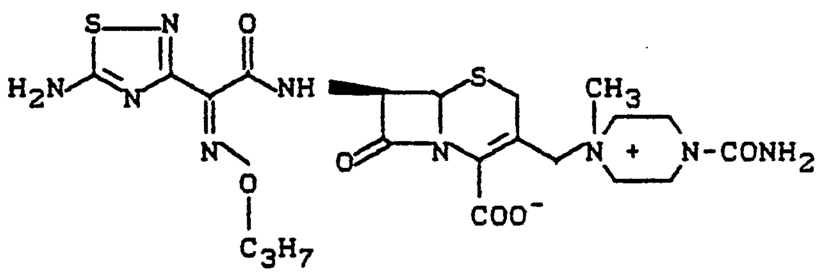

1,72 g (7,5 mmol) 2-(5-Amino-1,2,4-thiadiazol-3-yl)-2-(Z)-propyloxyiminoessigsäure werden mit 2,3 g (5 mmol) 7-Amino-3-(4-Aminocarbonyl-1-methylpiperazinium)methyl-3-cephem-4-carboxylat (x $H_2SO_4$) wie für Beispiel 2 beschrieben umgesezt zu 720 mg Produkt.
$^1$H-NMR (DCOOD): δ = 6,09 [1]d; 5,50 [1]d, 5,08 [1]bd, 4,48 [1]bd; 4,38 [2]t; 4,25 -3,60 [10]m; 3,38 [3]s; 1,80 [2]sex.; 0,95 [3]t ppm.

Beispiel 4

7β-[2-(5-Amio-1.2,4.-thiadiazol-3-yl)-2-(Z)-butyloxyiminoacetamido]-3-(4-aminocarbonyl-1-methylpiperazinium)-methyl-3-cephem-4-carboxylat

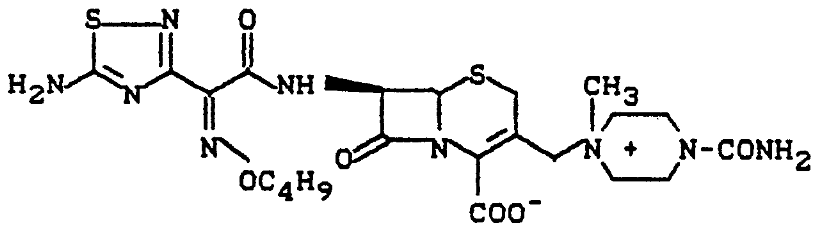

4,3 g (17,6 mmol) 2-(5-Amino-1,2,4-thiadiazol-3-yl)-2-(Z)-butyloxyiminoessigsäure werden mit 5,4 g (11,7 mmol) 7-Amino-3-(4-aminocarbonyl-1-methylpiperazinium)methyl-3-cephem-4-carboxylat (x $H_2SO_4$) wie für Beispiel 2 beschrieben umgesetzt zu 1,55 g Produkt.
$^1$H-NMR (DCOOD): δ = 6,08 [1]d; 5,48 [1]d; 5,08 [1]bd; 4,48 [1]bd; 4,41 [2]t; 4,25 - 3,63 [10]m; 3,36 [3]s; 1,75 [2]quin.; 1,40 [2]sex; 1,90 [3]t ppm.

Beispiel 5

7β-[2-(5-Amino-1,2,4-thiadiazol-3-yl)-2-(Z)-allyloxyiminoacetamido]-3-(4-aminocarbonyl-1-methylpiperazinium)methyl-3-cephem-4-carboxylat

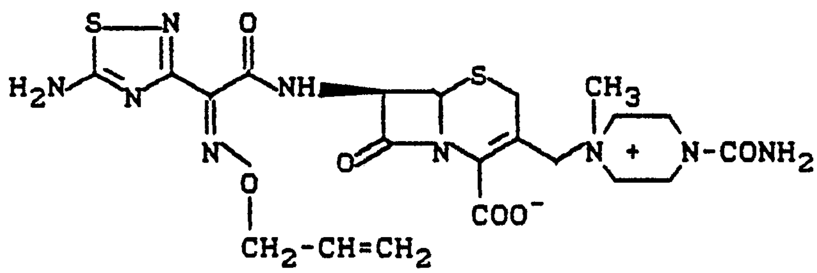

3,44 g (15 mmol) 2-(5-Amino-1,2,4-thiadiazol-3-yl)-2-(Z)-allyloxyiminoessigsäure werden mit 3,4 g (7,5 mmol) 7-Amino-3-(4-aminocarbonyl-1-methylpiperazinium)methyl-3-cephem-4-carboxylat (x $H_2SO_4$) wie für Beispiel 2 beschrieben umgesetzt zu 700 mg Produkt.
$^1$H-NMR (DCOOD): δ = 6.06 [1]d; 5,45 [1)d; 5,30 [1]bd; 5,06 - 4,75 [2]m; 4,47 [1]bd; 4,25 - 3,55 [4]m, 3,38 - 3,13 [13]m ppm.

Beispiel 6

7β-[2-(5-Amino-1,2,4-thiadiazol-3-yl)-2-(Z)-cyclopentyloxyacetamido]-3-(4-aminocarbonyl-1-methyl-piperazinium)methyl-3-cephem-4-carboxylat

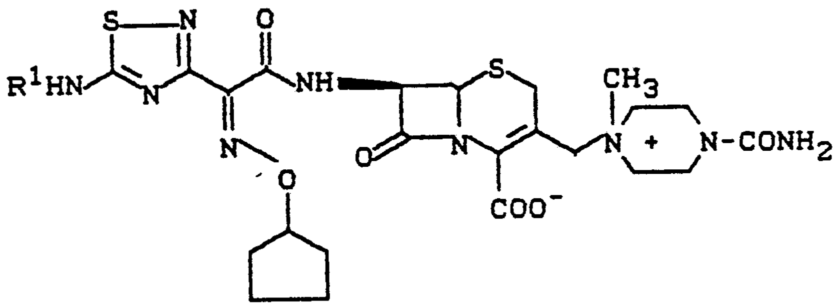

3,3 g (12,9 mmol) 2-(5-Amino-1,2,4-thiadiazol-3-yl)-2-(Z)-cyclopentyloxyiminoessigsäure werden mit 3,0 g (6,5 mmol) 7-Amino-3-(4-aminocarbonyl-1-methylpiperazinium)methyl-3-cephem-4-carboxylat (x $H_2SO_4$) wie für Beispiel 2 beschrieben umgesetzt zu 770 mg Produkt.
$^1$H-NMR (DCOOD): δ = 6.08 [1]d; 5,49 [1]d; 5,08 [1]bd; 5,00 [1]quin; 4,48 [1]bd; 4,3 - 3,6 [10]m; 3,38 [3]s; 2,0 - 1,5 [8]m ppm.

**Ansprüche**

1. Cephalosporinderivate der allgemeinen Formel (I)

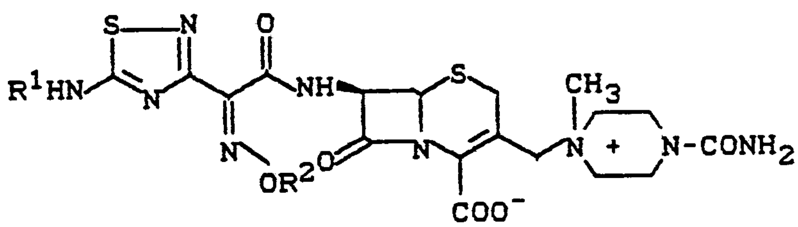

(I)

in welcher
$R^1$ -für Wasserstoff oder
-für eine Aminoschutzgruppe steht
und
$R^2$ -für Alkyl,
-für Cycloalkyl, oder
-für Alkenyl steht.

2. Cephalosporinderivate der Formel (I) nach Anspruch 1, in der Alkyl für einen geradkettigen oder verzweigten Kohlenwasserstoffrest mit 1 bis 12 Kohlenstoffatomen und Alkenyl für einen geradkettigen oder verzweigten Kohlenwasserstoffrest mit 2 bis 12 Kohlenstoffatomen steht.

3. Cephalosporinderivate der allgemeinen Formel (I) nach Anspruch 1, in welcher
$R^1$ -für Wasserstoff, oder
-für Acetyl steht
und
$R^2$ -für geradkettiges oder verzweigtes Alkyl mit bis zu 8 Kohlenstoffatomen steht, oder
-für Cyclopropyl, Cyclopentyl oder Cyclohexyl steht, oder
- für geradkettiges oder verzweigtes Alkenyl mit bis zu 8 Kohlenstoffatomen und ein oder zwei Doppelbindungen steht.

4. Cephalosporinderivate der allgemeinen Formel (I) nach Anspruch 1, in welcher
$R^1$ -für Wasserstoff steht
und
$R^2$ -für geradkettiges oder verzweigtes Alkyl mit bis zu 6 Kohlenstoffatomen,

-für Cyclopentyl, Cyclohexyl oder

-für geradkettiges oder verzweigtes Alkenyl mit bis zu 4 Kohlenstoffatomen und einer Doppelbindung steht.

5. Cephalosporinderivate der allgemeinen Formel (I) nach Anspruch 1, in welcher $R^2$ -für Methyl, Ethyl, Propyl, Isopropyl, Butyl, Isobutyl, Pentyl, Isopentyl, Hexyl, Isohexyl, Heptyl, Isoheptyl, Octyl, Isooctyl, Allyl, Propenyl, Isopropenyl, Butenyl, Isobutenyl, Pentenyl, Isopentenyl, Hexenyl, Isohexenyl, Heptenyl, Isoheptenyl, Octenyl oder Isooctenyl steht.

6. Verfahren zur Herstellung der Cephalosporinderivate der allgemeinen Formel (I) nach Anspruch 1, dadurch gekennzeichnet, daß man Carbonsäuren der allgemeinen Formel (II)

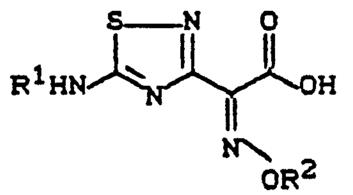

(II)

in welcher

$R^1$ und $R^2$ die oben angegebene Bedeutung haben,

nach Aktivierung der Carboxylgruppe durch Überführung in ein gemischtes Anhydrid oder durch Überführen in das Säurehalogenid oder durch Überführen in einen aktivierten Ester mit der $\beta$-Lactam-Verbindung der Formel (III)

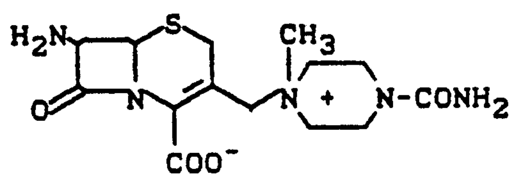

(III)

umsetzt, dann gegebenenfalls Schutzgruppen abspaltet und die gewünschten Salze oder aus Salzen die freien Säuren herstellt.

7. Verfahren zur Herstellung der Cephalosporinderivate der allgemeinen Formel (I) nach Anspruch 1, dadurch gekennzeichnet, daß man Ester der allgemeinen Formel (VI)

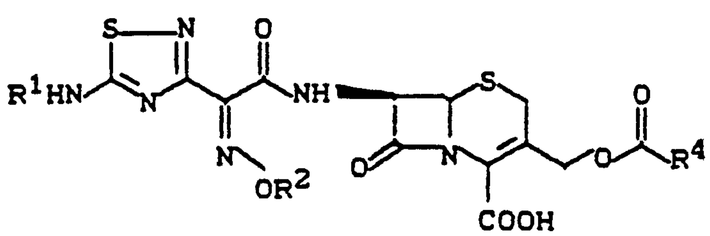

(VI)

in welcher

$R^1$ und $R^2$ die angegebene Bedeutung haben

und

$R^4$ -für gegebenenfalls substituiertes Alkyl oderAryl steht, bevorzugt für Methyl, Ethyl, Propyl, Chlormethyl, Dichlormethyl, Trichlormethyl, Trifluormethyl oder Phenyl steht, oder besonders bevorzugt für eine Methylgruppe steht,

silyliert,

die Silylverbindung der allgemeinen Formel (VII)

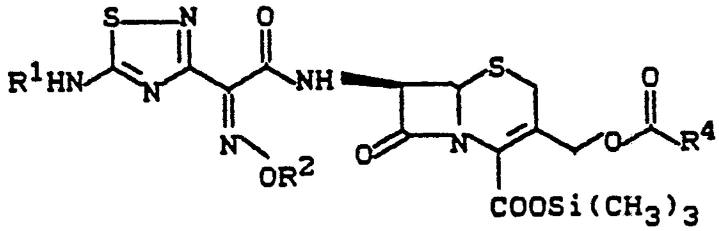

(VII)

in welcher
$R^1$, $R^2$ und $R^4$ die oben angegebene Bedeutung haben,
in die Iodmethylverbindungen der allgemeinen Formel (VIII)

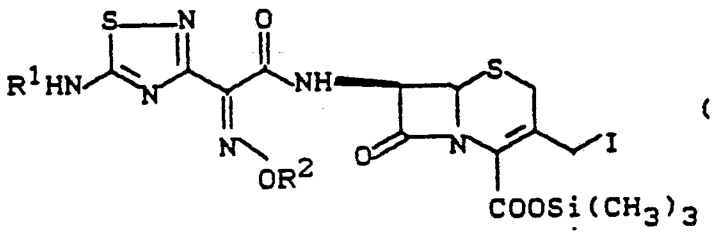

(VIII)

in welcher
$R^1$ und $R^2$ die oben angegebene Bedeutung haben,
überführt
und diese anschließend mit 1-Aminocarbonyl-4-methylpiperazin der Formel (IX)

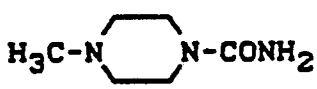

(IX)

umsetzt.

8. Cephalosporinderivate der allgemeinen Formel (I)

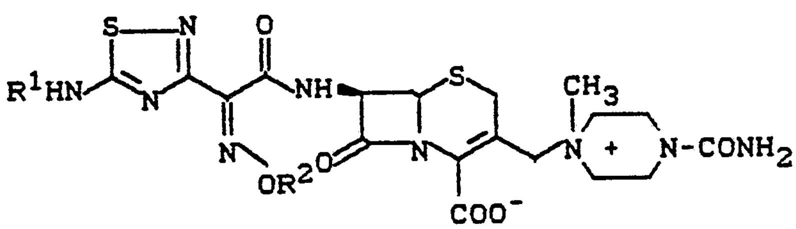

(I)

in welcher
$R^1$ -für Wasserstoff oder
-für eine Aminoschutzgruppe steht
und
$R^2$ -für Alkyl,
-für Cycloalkyl, oder
-für Alkenyl steht,
zur Verwendung in einem Verfahren zur therapeutischen Behandlung des menschlichen oder tierischen Körpers.

9. Arzneimittel, enthaltend Cephalosporinderivate der allgemeinen Formel (I)

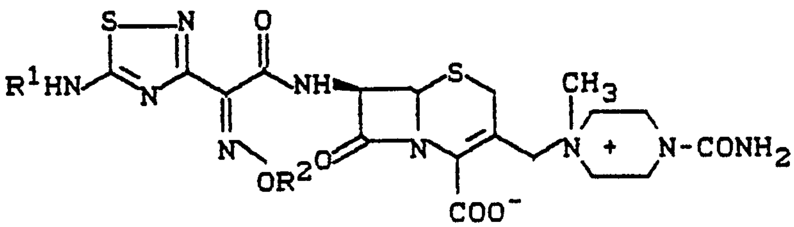

(I)

in welcher
R' -für Wasserstoff oder
-für eine Aminoschutzgruppe steht
und
$R^2$ -für Alkyl,
-für Cycloalkyl, oder
-für Alkenyl steht.

10. Verwendung von Cephalosporinderivaten der allgemeinen Formel (I)

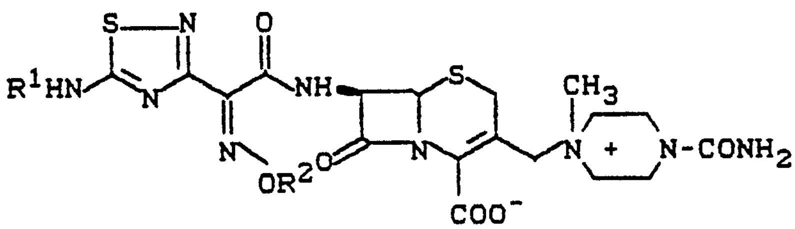

(I)

in welcher
R' -für Wasserstoff oder
-für eine Aminoschutzgruppe steht
und
$R^2$ -für Alkyl,
-für Cycloalkyl, oder
-für Alkenyl steht,
zur Herstellung von Arzneimitteln.